**Europäisches Patentamt**

**European Patent Office**

**Office européen des brevets**

(19)

(11) Veröffentlichungsnummer: **0 317 909 B1**

## EUROPÄISCHE PATENTSCHRIFT

(12)

(45) Veröffentlichungstag der Patentschrift: **20.01.93**

(51) Int. Cl.⁵: **C07C 45/75**, C07C 47/22, C07C 47/21

(21) Anmeldenummer: **88119187.8**

(22) Anmeldetag: **18.11.88**

(54) **Verfahren zur Herstellung von alpha-Alkylacroleinen.**

(30) Priorität: **27.11.87 DE 3740293**

(43) Veröffentlichungstag der Anmeldung:
**31.05.89 Patentblatt 89/22**

(45) Bekanntmachung des Hinweises auf die
Patenterteilung:
**20.01.93 Patentblatt 93/03**

(84) Benannte Vertragsstaaten:
**AT BE CH DE ES FR GB IT LI NL SE**

(56) Entgegenhaltungen:
**EP-A- 0 058 927**
**EP-A- 0 092 097**
**FR-A- 2 444 659**
**GB-A- 2 078 748**

(73) Patentinhaber: **HOECHST AKTIENGESELL-SCHAFT**
**Postfach 80 03 20**
**W-6230 Frankfurt am Main 80(DE)**

(72) Erfinder: **Diekhaus, Gerhard, Dr.Dipl.Chem.**
**Walsumermarkstrasse 89**
**W-4200 Oberhausen 11(DE)**
Erfinder: **Kappesser, Harald**
**Waidmannsweg 24**
**W-4200 Oberhausen 11(DE)**

EP 0 317 909 B1

**Beschreibung**

Die vorliegende Erfindung betrifft ein verbessertes Verfahren zur Herstellung von alpha-Alkylacroleinen durch Umsetzung von Aldehyden der allgemeinen Formel $R.CH_2.CHO$ und Formaldehyd.

Für die Herstellung von Methacrolein sind zahlreiche Verfahren bekannt. Unter ihnen haben, wegen der leichten Zugänglichkeit der Ausgangsstoffe, in letzter Zeit diejenigen an Bedeutung gewonnen, die von Propionaldehyd ausgehen.

So erhält man durch Kondensation der Ausgangsaldehyde an Natriumoxid und Kieselsäure enthaltenden Katalysatoren bei 275°C Methacrolein in einer Ausbeute von etwa 46 % (vgl. C.A. Bd. 56 [1962], Spalten 2321 und 2322).

In der DE-C1 875 194 ist die Herstellung von Methacrolein beschrieben, wobei man zu einem Gemisch aus wäßriger Formaldehydlösung Propionaldehyd, Natriumchlorid und Buttersäure bei Siedetemperatur im Laufe von 2 bis 3 Stunden Piperidin zutropft. Nach weiteren 3 Stunden Nachreaktion soll Methacrolein in 95 %iger Ausbeute erhälten werden. Abgesehen davon, daß lange Reaktionszeiten erforderlich sind, konnte beim Nacharbeiten der Vorschrift die hohe Ausbeute bei weitem nicht erreicht werden.

Nach der Lehre der DE-B2 28 55 504 setzt man zur Herstellung von Methacrolein Propionaldehyd mit Formaldehyd in Gegenwart eines Katalysatorsystems um, das aus einem sekundären Amin und einer Carbonsäure mit bis zu 5 Kohlenstoffatomen besteht und läßt bei Temperaturen von 70 bis 120°C und Drücken von 2 bis 10 bar reagieren. Das Verfahren liefert Methacrolein-Ausbeuten von 80 bis 82 %.

In der DE-A1-30 25 350 ist die Erweiterung des vorstehend erläuterten Verfahrens allgemein auf die Herstellung von 2-Methylenaldehyden beschrieben.

Gegenstand der DE-A1 31 06 557 ist ein Verfahren zur Herstellung von alpha-Alkylacroleinen durch Umsetzung von Alkanalen mit Formaldehyd und sekundärem Amin in Gegenwart von Säure. Die Reaktion erfolgt in saurem bis neutralem Milieu bei pH-Werten von 2,5 bis 7. Nach den Beispielen erhält man Methacrolein zwar in hohen Ausbeuten von 90 und mehr %, doch erfordert der Prozeß einen sehr hohen Amineinsatz von 0,5 bis 1 Äquivalent je Mol Alkanal.

Die DE-A1 32 13 681 betrifft ebenfalls ein Verfahren zur Herstellung von alpha-Alkylacroleinen aus Alkanalen und Formaldehyd in Gegenwart von sekundären Aminen und gewünschtenfalls von Säuren. Es ist dadurch gekennzeichnet, daß die Umsetzung unter Überdruck bei einer Temperatur von mehr als 150°C und einer Reaktionszeit von höchstens 25 Minuten durchgeführt wird. Auch dieses Verfahren liefert Methacrolein in hohen Ausbeuten, erfordert aber die Anwendung von vergleichsweise hohen Temperaturen und Drücken.

Es bestand daher die Aufgabe, eine Arbeitsweise bereitzustellen, die alpha-Alkylacroleine in hohen Ausbeuten unter möglichst milden Reaktionsbedingungen liefert.

Diese Aufgabe wird gelöst durch ein Verfahren zur Herstellung von alpha-Alkylacroleinen aus Aldehyden der allgemeinen Formel $R.CH_2.CHO$, wobei R ein geradkettiger oder verzweigter Alkylrest mit 1 bis 10 Kohlenstoffatomen ist, und Formaldehyd in Gegenwart von 0,02 bis 0,05 Mol eines sekundären Amins und 0,02 bis 0,065 Mol einer Carbonsäure mit bis zu 5 Kohlenstoffatomen je Mol des Aldehyds $R.CH_2.CHO$ bei 70 bis 120°C und 0,2 bis 1 MPa, dadurch gekennzeichnet, daß das Molverhältnis von Amin zu Carbonsäure 1 : 0,8 bis 1 : 1,3 beträgt und die Reaktion in zwei Stufen durchgeführt wird, wobei in der ersten Stufe der Aldehyd $R.CH_2.CHO$ und Formaldehyd in Gegenwart des sekundären Amins und 25 bis 75 Mol-% der Carbonsäure umgesetzt werden und in der zweiten Stufe nach Zusatz der restlichen Carbonsäure die Reaktion zuende geführt wird.

Überraschenderweise führen die beanspruchten Maßnahmen in kurzer Zeit zu alpha-Alkylacroleinen in hoher Ausbeute, obgleich die Umsetzung bei mäßigen Temperaturen und nur leicht erhöhtem Druck durchgeführt wird.

Der Rest R in der allgemeinen Formel der als Ausgangsmaterial verwendeten Aldehyde steht für einen Alkylrest mit 1 bis 10 Kohlenstoffatomen. Dieser Rest kann geradkettig oder auch verzweigt sein. Beispiele für Aldehyde, die nach der erfindungsgemäßen Arbeitsweise zur Reaktion gebracht werden können, sind Propanal, n-Butanal, 3-Methylbutanal, n-Pentanal, n-Hexanal, 3-Methylhexanal, 4-Methylhexanal, n-Heptanal. Besonders bewährt hat sich das Verfahren für die Umsetzung von Propanal, n-Butanal, 3-Methylbutanal.

Formaldehyd wird im allgemeinen als wäßrige Lösung angewandt. Er kann aber auch in polymerisierter Form als Paraformaldehyd umgesetzt werden.

Aldehyd $R.CH_2.CHO$ und Formaldehyd können im stöchiometrischen Verhältnis zur Reaktion gebracht werden. Es ist aber auch möglich, einen der beide Aldehyde im Überschuß anzuwenden. Es hat sich bewährt, je Mol Aldehyd $R.CH_2.CHO$ 0,9 bis 1,5 Mol Formaldehyd einzusetzen.

Die Anwendung eines Lösungsmittels, abgesehen vom Wasser, in dem der Formaldehyd enthalten sein kann, ist nicht erforderlich, bei Einsatz einer polymeren Form des Formaldehyds jedoch zweckmäßig. Als Lösungsmittel geeignet sind Kohlenwasserstoffe und aliphatische Alkohole, z.B. Isodecan, Toluol und 2-Ethylhexanol.

Ein wesentliches Merkmal des beanspruchten Prozesses ist die Verwendung von sekundären Aminen und Carbonsäuren, beide wirken zusammen als Katalysator, in bestimmten Molverhältnissen zueinander und zum Aldehyd. Je Mol Aldehyd setzt man 0,02 bis 0,05, vorzugsweise 0,025 bis 0,035 eines sekundären Amins und 0,02 bis 0,065 Mol, vorzugsweise 0,025 bis 0,040 einer Carbonsäure mit bis zu 5 Kohlenstoffatomen ein. Weiterhin ist für das neue Verfahren kennzeichnend, daß das Molverhältnis von Amin zu Carbonsäure 1 : 0,8 bis 1,3 beträgt. Bevorzugt setzt man Amin und Carbonsäure im Molverhältnis 1 : 1 und insbesondere im Molverhältnis 1 : 1,1 bis 1,3 ein.

Als sekundäre Amine kann man sowohl nieder- als auch höhermolekulare, aliphatische Verbindungen einsetzen. Die Alkylreste können gleich oder verschieden sein. Beispiele für geeignete sekundäre Amine sind Dipropylamin, Methylbutylamin, Ethylbutylamin oder Di-n-octylamin. Besonders bewährt hat sich Di-n-butylamin. Die sekundären Amine brauchen nicht als einheitliche Substanz eingesetzt zu werden, auch Mischungen der Isomeren desselben Amins oder Gemische verschiedener Amine haben sich als gut geeignet zur Durchführung der Reaktion erwiesen.

Die Carbonsäuren mit bis zu 5 Kohlenstoffatomen können eine, aber auch mehrere Carboxylgruppen enthalten. Bewährt haben sich Ameisensäure, Essigsäure und insbesondere Propionsäure, Buttersäure oder Valeriansäure, die als solche oder in Mischungen eingesetzt werden.

Ein weiteres sehr wichtiges Merkmal der erfindungsgemäßen Arbeitsweise ist die Durchführung der Umsetzung in zwei Stufen. Hierbei werden Aldehyd und Formaldehyd zunächst in Gegenwart des gesamten sekundären Amins mit einem Teil der Carbonsäure zur Reaktion gebracht. Anschließend setzt man die restliche Carbonsäure zu. In der ersten Stufe gibt man 25 bis 75, vorzugsweise 30 bis 65 Mol-% Carbonsäure dem Reaktionsgemisch zu, in der zweiten Stufe den Rest. Beide Stufen der Reaktion führt man zweckmäßig im selben Reaktor durch. Es war nicht vorauszusehen, daß der abgestufte Säurezusatz, der bedeutet, daß die Umsetzung in unterschiedlichen pH-Bereichen erfolgt, zu einer merklichen Ausbeute-Erhöhung führt.

Üblicherweise verläuft die Reaktion in der Flüssigphase unter Einhaltung eines Reaktionsdruckes zwischen 0,2 und 1 MPa, vorzugsweise 0,2 bis 0,4 MPa. Es ist aber auch möglich, die Umsetzung in der Gasphase vorzunehmen. Die Reaktionstemperatur beträgt zwischen 70 und 120°C, 95 bis 110°C werden bevorzugt.

Bei Umsetzung der Reaktanten in der flüssigen Phase arbeitet man in einem Druckgefäß, in das man unter Stickstoffatmosphäre den Aldehyd und Formaldehyd und gegebenenfalls den Teil der Carbonsäure vorlegt, der in der ersten Stufe eingesetzt wird. Anschließend fügt man zweckmäßig unter intensiver Rührung das sekundäre Amin zu. Es ist auch möglich, Amin und Carbonsäure in das vorgelegte Aldehydgemisch einzutragen. Falls erforderlich, muß das Reaktionsgemisch gekühlt werden, um innerhalb des Bereiches der Reaktionstemperatur zu bleiben. Man kann aber auch durch anteilsweisen Zusatz der Reaktanten sicherstellen, daß eine Überschreitung der Reaktionstemperatur vermieden wird. Nach Zugabe des gesamten Amins läßt man 20 bis 60 Minuten reagieren und gibt darauf, ebenfalls unter intensiver Mischung, die restliche Carbonsäure hinzu. Gegebenenfalls ist das Reaktionsgemisch nunmehr zu erhitzen, um die Umsetzungstemperatur zu erreichen. Nach weiteren 20 bis 60 Minuten ist die Reaktion beendet. Man läßt das Reaktionsgemisch abkühlen, wobei es sich in eine organische und eine wäßrige Phase trennt. Die neue Arbeitsweise kann nicht nur diskontinuierlich, sondern auch kontinuierlich, z.B. in einer zweistufigen Rührwerkskaskade durchgeführt werden. Aus dem Rohprodukt wird das alpha-Alkylacrolein durch fraktionierte Destillation in mehr als 99%iger Reinheit erhalten. Fur die meisten Verwendungszwecke ist eine zusätzliche Reinigung nicht erforderlich.

Das erfindungsgemäße Verfahren erlaubt die Herstellung von alpha-Alkylacroleinen bei niedrigen Temperaturen und unter solchen Bedingungen, die keine Spezialapparaturen erfordern. Bemerkenswert ist die hohe Ausbeute an ungesättigtem Aldehyd, die trotz der einfachen Reaktionsführung erzielt wird.

In den folgenden Beispielen wird das erfindungsgemäße Verfahren näher beschrieben. Selbstverständlich ist nicht beabsichtigt, die Erfindung auf die speziellen Ausführungsformen zu beschränken.

Beispiel

In einem mit Rührer ausgerüsteten Druckbehälter von 0,6 m$^3$ Inhalt werden Aldehyd R.CH$_2$.CHO und Formaldehyd (in Form einer 30 %igen wäßrigen Lösung) und ein Teil der Carbonsäure in den der Tabelle zu entnehmenden Mengen unter Stickstoffatomosphäre gemischt. Innerhalb von 30 Minuten setzt man das gesamte Di-n-butylamin zu. Anschließend wird das Reaktionsgemisch innerhalb weiterer 30 Minuten auf 95 bis 100°C erhitzt. Nach Zugabe der restlichen Carbonsäure läßt man, gegebenenfalls unter Rühren, 30 Minuten nachreagieren. Das Reaktionsgemisch wird abgekühlt, die wäßrige Phase abgetrennt. Durch gaschromatographische Analyse wird die Zusammensetzung der organischen Phase bestimmt.

**Tabelle 1: <u>Herstellung von 2-Methylacrolein</u>**

|  | | Beispiel 1 (Vergleich) | Beispiel 2 |
|---|---|---|---|
| Propionaldehyd (kMol) | | 1,8 | 2,16 |
| Formaldehyd (kMol) | | 1,98 | 2,38 |
| Di-n-butylamin (kMol) | | 0,045 | 0,054 |
| kMol Amin/kMol Propionaldehyd | | 0,025 | 0,025 |
| Propionsäure (kMol) | | 0,027 | 0,054 |
| 1. Stufe | | 0,027 | 0,032 |
| 2. Stufe | | – | 0,022 |
| kMol Säure/kMol Propionaldehyd | | 0,015 | 0,025 |
| kMol Säure/kMol Amin | | 0,6 | 1,0 |
| GC-Analyse d.org.Phase | | | |
| 2-Methylacrolein | (Gew.-%) | 92,7 | 92,25 |
| Aldol | (Gew.-%) | 5,6 | 6,0 |
| Nachlauf | (Gew.-%) | 1,5 | |
| Ausbeute | (% d.Th.) | 81,7 | 89,0 |

Tabelle 2: Herstellung von 2-Ethylacrolein

| | Beispiel 3 (Vergleich) | Beispiel 4 (Vergleich) | Beispiel 5 | Beispiel 6 |
|---|---|---|---|---|
| n-Butyraldehyd (kMol) | 1,75 | 2,11 | 1,75 | 1,75 |
| Formaldehyd (kMol) | 1,75 | 2,11 | 1,75 | 1,75 |
| Di-n-butylamin (kMol) | 0,0446 | 0,0549 | 0,0446 | 0,0446 |
| kMol Amin/kMol Butyraldehyd | 0,026 | 0,026 | 0,025 | 0,025 |
| n-Buttersäure (kMol) 1. Stufe | 0,014 | 0,055 | 0,055 | 0,055 |
| 2. Stufe | 0,014 | 0,055 | 0,018 | 0,0275 |
| | — | — | 0,037 | 0,0275 |
| kMol Säure/kMol Butyraldehyd | 0,008 | 0,026 | 0,03 | 0,03 |
| kMol Säure/kMol Amin | 0,3 | 1,0 | 1,2 | 1,2 |
| GC-Analyse d. org. Phase 2-Ethylacrolein (Gew.-%) | 91,6 | 84,9 | 92,6 | 90,4 |
| Aldol (Gew.-%) | 3,9 | 5,5 | 1,4 | 0,2 |
| Nachlauf (Gew.-%) | 4,5 | 3,2 | 0,4 | 0,5 |
| Ausbeute (% d. Th.) | 84,5 | 76,7 | 97,4 | 98,4 |

**Patentansprüche**

1. Verfahren zur Herstellung von alpha-Alkylacroleinen aus Aldehyden der allgemeinen Formel R.CH$_2$.CHO, wobei R ein geradkettiger oder verzweigter Alkylrest mit 1 bis 10 Kohlenstoffatomen ist, und Formaldehyd in Gegenwart von 0,02 bis 0,05 Mol eines sekundären Amins und 0,02 bis 0,065 Mol einer Carbonsäure mit bis zu 5 Kohlenstoffatomen je Mol des Aldehyds $R.CH_2.CHO$ bei 70 bis 120°C und 0,2 bis 1 MPa, dadurch gekennzeichnet, daß das Molverhältnis von Amin zu Carbonsäure 1 : 0,8 bis 1 : 1,3 beträgt und die Reaktion in zwei Stufen durchgeführt wird, wobei in der ersten Stufe der Aldehyd $R.CH_2.CHO$ und Formaldehyd in Gegenwart des sekundären Amins und 25 bis 75 Mol-% der Carbonsäure umgesetzt werden und in der zweiten Stufe nach Zusatz der restlichen Carbonsäure die Reaktion zuende geführt wird.

2. Verfahren nach Anspruch 1, dadurch gekennzeichnet, daß je Mol Aldehyd 0,025 bis 0,035 Mol eines sekundären Amins eingesetzt werden.

3. Verfahren nach Anspruch 1 oder 2, dadurch gekennzeichnet, daß je Mol Aldehyd 0,025 bis 0,04 Mol einer Carbonsäure mit bis zu 5 Kohlenstoffatomen eingesetzt werden.

4. Verfahren nach einem oder mehreren der Ansprüche 1 bis 3, dadurch gekennzeichnet, daß das Molverhältnis von Amin zu Carbonsäure 1 : 1 und insbesondere 1 : 1,1 bis 1,3 beträgt.

5. Verfahren nach einem oder mehreren der Ansprüche 1 bis 4, dadurch gekennzeichnet, daß man in der ersten Stufe dem Reaktionsgemisch 30 bis 65 Mol-% Carbonsäure zusetzt.

6. Verfahren nach einem oder mehreren der Ansprüche 1 bis 5, dadurch gekennzeichnet, daß als sekundäres Amin Di-n-butylamin verwendet wird.

7. Verfahren nach einem oder mehreren der Ansprüche 1 bis 6, dadurch gekennzeichnet, daß als Carbonsäure Propionsäure, Buttersäure oder Valeriansäure verwendet wird.

## Claims

1. A process for the preparation of alpha-alkylacroleins from aldehydes with the general formula $R.CH_2.CHO$, R being a straight-chain or branched alkyl group having 1 to 10 carbon atoms, and formaldehyde in the presence of 0.02 to 0.05 moles of a secondary amine and 0.02 to 0.065 moles of a carboxylic acid having up to 5 carbon atoms per mole of aldehyde $R.CH_2.CHO$ at 70 to 120°C and 0.2 to 1 MPa, characterised in that the molar ratio of amine to carboxylic acid is 1 : 0.8 to 1 : 1.3 and the reaction is performed in two stages, the aldehyde $R.CH_2.CHO$ and formaldehyde being reacted in the first stage in the presence of the secondary amine and 25 to 75 mole % of the carboxylic acid and the reaction being completed in the second stage after the remaining carboxylic acid has been added.

2. A process according to claim 1, characterised in that 0.025 to 0.035 moles of a secondary amine are added per mole of aldehyde.

3. A process according to claim 1 or 2, characterised in that 0.025 to 0.04 moles of a carboxylic acid having up to 5 carbon atoms are added per mole of aldehyde.

4. A process according to one or more of the claims 1 to 3, characterised in that the molar ratio of amine to carboxylic acid is 1 : 1 and in particular 1 : 1.1 to 1.3.

5. A process according to one or more of claims 1 to 4, characterised in that 30 to 65 mole % of carboxylic acid is added to the reaction mixture in the first stage.

6. A process according to one or more of the claims 1 to 5, characterised in that di-n-butylamine is used as the secondary amine.

7. A process according to one or more of the claims 1 to 6, characterised in that propionic acid, butyric acid or valeric acid is used as carboxylic acid.

## Revendications

1. Procédé de préparation d'alpha-alkylacroléines à partir d'aldéhydes de formule générale $R.CH_2.CHO$,

**EP 0 317 909 B1**

dans laquelle R représente un groupe alkyle à chaîne droite ou ramifiée en C1-C10, et du formaldéhyde, en présence de 0,02 à 0,05 mol d'une amine secondaire et 0,02 à 0,065 mol d'un acide carboxylique contenant jusqu'à 5 atomes de carbone, dans les deux cas pour une mode de l'aldéhyde $R.CH_2.CHO$, à des températures de 70 à 120°C et des pressions de 0,2 à 1 MPa, caractérisé en ce que le rapport molaire amine/acide carboxylique est de 1:0,8 à 1:1,3 et en ce que la réaction est effectuée en deux stades opératoires : au premier stade, on fait réagir l'aldéhyde $R.CH_2.CHO$ et le formaldéhyde en présence de l'amine secondaire et de 25 à 75 mol % de l'acide carboxylique et au deuxième stade, après addition du complément d'acide carboxylique, on achève la réaction.

2.  Procédé selon la revendication 1, caractérisé en ce que l'on utilise de 0,025 à 0,035 mol d'une amine secondaire par mole d'aldéhyde.

3.  Procédé selon la revendication 1 ou 2, caractérisé en ce que l'on utilise de 0,025 à 0,4 mol d'un acide carboxylique contenant jusqu'à 5 atomes de carbone par mole d'aldéhyde.

4.  Procédé selon une ou plusieurs des revendications 1 à 3, caractérisé en ce que le rapport molaire amine/acide carboxylique est de 1:1 et plus spécialement de 1:1,1 à 1,3.

5.  Procédé selon une ou plusieurs des revendications 1 à 4, caractérisé en ce que, au premier stade opératoire, on ajoute au mélange de réaction de 30 à 65 mol % de l'acide carboxylique.

6.  Procédé selon une ou plusieurs des revendications 1 à 5, caractérisé en ce que l'amine secondaire utilisée est la di-n-butylamine.

7.  Procédé selon une ou plusieurs des revendications 1 à 6, caractérisé en ce que l'acide carboxylique utilisé est l'acide propionique, l'acide butyrique ou l'acide valérique.

7